# EUROPEAN PATENT SPECIFICATION

(11) **EP 1 844 143 B1**
(45) Date of publication and mention of the grant of the patent: **17.06.2009**
(21) Application number: 05707166.4
(22) Date of filing: 03.02.2005
(51) Int. Cl.: C12N 15/10, C12N 15/11, C12Q 1/68, G01N 33/50

(54) **METHOD OF GENERATING TRANSLATIONALLY ACTIVE LINEAR DNA MOLECULES AND USE THEREOF IN ARRAY FORMATS**
VERFAHREN ZUR HERSTELLUNG TRANSKRIPTIONSAKTIVER LINEARER DNA-MOLEKÜLE UND DEREN VERWENDUNG AUF ARRAYS
PROCEDE PERMETTANT DE PRODUIRE DES MOLECULES D'ADN LINEAIRE ACTIVES SUR AU NIVEAU TRADUCTIONEL ET LEUR EMPLOI DANS UNE STRUCTURE DE JEU ORDONNE

(43) Date of publication of application: 17.10.2007
(73) Proprietor: King Faisal Specialist Hospital & Research Centre, Riyadh 11211 (SA)
(72) Inventor: ABU KHABAR, Khalid S., Riyadh 11211 (SA)
(74) Representative: Engelhard, Markus
(86) International application number: PCT/EP2005/001078
(87) International publication number: WO 2006/081831

(56) References cited:
- WO-A-01/20015
- US-A- 5 571 690
- LI S ET AL: "Delivery of a PCR amplified DNA fragment into cells: A model for using synthetic genes for gene therapy" GENE THERAPY, vol. 4, no. 5, 1997, pages 449-454, XP002345971 ISSN: 0969-7128
- HOFMAN C R ET AL: "Efficient in vivo gene transfer by PCR amplified fragment with reduced inflammatory activity" GENE THERAPY, vol. 8, no. 1, January 2001 (2001-01), pages 71-74, XP002345972 ISSN: 0969-7128
- CASTANOTTO D ET AL: "Functional siRNA expression from transfected PCR products" RNA, CAMBRIDGE UNIVERSITY PRESS, CAMBRIDGE, GB, vol. 8, no. 11, November 2002 (2002-11), pages 1454-1460, XP002281435 ISSN: 1355-8382
- GOU D ET AL: "Gene silencing in mammalian cells by PCR-based short hairpin RNA" FEBS LETTERS, ELSEVIER SCIENCE PUBLISHERS, AMSTERDAM, NL, vol. 548, no. 1-3, 31 July 2003 (2003-07-31), pages 113-118, XP004441071 ISSN: 0014-5793
- SCHAKOWSKI F ET AL: "A NOVEL MINIMAL-SIZE VECTOR (MIDGE) IMPROVES TRANSGENE EXPRESSION IN COLON CARCINOMA CELLS AND AVOIDS TRANSFECTION OF UNDESIRED DNA" MOLECULAR THERAPY, ACADEMIC PRESS, SAN DIEGO, CA,, US, vol. 3, no. 5, PART 1, May 2001 (2001-05), pages 793-800, XP001100620 ISSN: 1525-0016
- TAKI M ET AL: "A DIRECT AND EFFICIENT SYNTHESIS METHOD FOR DUMBELL-SHAPED LINEAR DNA USING PCR IN VITRO" NUCLEIC ACIDS RESEARCH, OXFORD UNIVERSITY PRESS, SURREY, GB, no. 3/30th SYMP, 17 September 2003 (2003-09-17), pages 191-192, XP002981766 ISSN: 0305-1048
- ZIAUDDIN J ET AL: "Microarrays of cells expressing defined cDNAs" NATURE, NATURE PUBLISHING GROUP, LONDON, GB, vol. 411, no. 6833, 3 May 2001 (2001-05-03), pages 107-110, XP002246363 ISSN: 0028-0836
- NECHANSKY ANDREAS ET AL: "In vitro expression of PCR fragments: A convenient tool for generating purified recombinant proteins in analytical amounts" METHODS IN MOLECULAR AND CELLULAR BIOLOGY, vol. 6, no. 2, 1995, pages 94-103, XP009054174 ISSN: 0898-7750
- RESTO ET AL: "AMPLIFICATION OF PROTEIN EXPRESSION IN A CELL FREE SYSTEM" NUCLEIC ACIDS RESEARCH, OXFORD UNIVERSITY PRESS, SURREY, GB, vol. 20, no. 22, 1992, pages 5979-5983, XP002119012 ISSN: 0305-1048

## Description

### SUMMARY

This invention describes a method for producing expressionally and translationally active linear DNA molecules that can be used as protein expression cassettes and are useful for high throughput protein expression and analysis in living cells. The method comprises a PCR amplification using two primers complementary to the sequences flanking the DNA sequence of interest, such as a cDNA, an open reading frame or a gene, that exists in expression vectors. The resultant PCR product contains a promoter, the DNA sequence of interest, and a termination sequence. The invention further provides different uses of these translationally active DNA cassettes, such as in proteome arrays.

### FIELD OF INVENTION

The present invention relates to a method for producing expressionally and translationally active linear DNA molecules. The field of the invention lies in functional proteomics, transfection and expression research tools, high-throughput analysis of gene function in mammalian cells and array-based proteomics.

### BACKGROUND OF THE INVENTION

A significant bottleneck arises from the challenge in R&D that most of the proteome, i.e. the proteins encoded by the human genome, is not known. This has led to missing many potentially useful targets for therapy. It is estimated that current therapies are only directed to 300 proteins. But, what about the rest of the proteome? The human genome contains approximately 30,000-50,000 genes, which harbor the information for over 500,000 different proteins. It is essential to know the function of the proteins so that new or better therapeutics can be achieved because as many as 10,000 proteins could be potential drug targets. These derive the need for high throughput analysis of protein function particularly for mammalian expression and in vitro transcription/translation systems.

Mammalian expression vectors are widespread tools to study the biological function of a protein. These vectors need to contain a strong mammalian promoter in order to express the gene downstream of the promoter. Among the most commonly used promoter, is the enhancer and promoter of the immediate early gene of human cytomegalovirus (hCMV). Irrespective of the cell line and of the state of the DNA, the hCMV enhancer-promoter was considerably stronger than both the SV40 promoter and the long terminal repeat of Rous sarcoma virus (RSV Foecking MK, Hofstetter H. (1986) Powerful and versatile enhancer-promoter unit for mammalian expression vectors. Gene 45(1): 101-5). These promoters are a target for RNA polymerase II which transcribes protein-encoding genes. The eukaryotic promoter, also called mammalian promoter, typically contains a TATAAA sequence (termed a TATA box) which is recognized by a protein termed TATA binding protein (TBP) that is associated with other transcription factors. These protein complexes recruit RNA polymerase II. Promoters may also contain enhancer elements. Many expression vectors contain these basal activated promoters, such as CMV, but may also contain regulated and inducible promoters, such as tetracycline or hormone regulated promoters.

There are some significant differences between prokaryotic and eukaryotic mRNA transcripts. Typically, eukaryotic mRNAs are characterized by two post-transcriptional modifications: a 5'-7 methyl-GTP cap and a 3' poly(A) tail. Both modifications contribute to the stability of the mRNA by preventing degradation. Additionally, the 5' cap structure enhances the translation of mRNA by helping to bind the eukaryotic ribosome and assuring recognition of the proper AUG initiator codon. This function may vary with the translation system and with the specific mRNA being synthesized. The consensus sequence 5'-GCCACCAUGG-3', also known as the "Kozak" sequence, is considered to be the strongest ribosomal binding signal in eukaryotic mRNA. An mRNA that lacks the Kozak consensus sequence may be translated efficiently in eukaryotic cell-free systems, if it possesses a moderately long 5'-untranslated region (UTR) that lacks stable secondary structure.

In bacteria, the ribosome is guided to the AUG initiation site by a purine-rich region called the Shine-Dalgarno (SD) sequence. This sequence is complementary to the 3' end of the 16s rRNA in the 30S ribosomal subunit. Upstream from the initiation AUG codon, the SD region has the consensus sequence 5'-UAAGGAGGUGA-3'. Specific mRNAs vary considerably in the number of nucleotides that complement the anti-Shine-Dalgarno sequence of 16S rRNA, ranging from as few as two to nine or more. The position of the ribosome-binding site (RBS) in relation to the AUG initiator is very important for efficiency of translation (usually from -6 to -10 relative to the A of the initiation site).

Unlike termination signals in bacteria, where the 3' end of the mRNA is formed by the RNA polymerase that is simply dropping of the DNA and ceasing transcription, in eukaryotes cleavage followed by the polyadenylation complex binding to an AAUAAA sequence near the end of the mRNA occurs. This complex contains an endonuclease that cuts the RNA about 30 bases downstream of the AAUAAA sequence and a polymerase that adds a string of poly A forming the poly A tail.

Thus, expression vectors have sequences incorporated that make the cDNA capable of being transcribed and translated into protein including ribosome binding sites and poly A sites.

There is a need for methods producing arrays or other assays containing the information needed to synthesize proteins either *in vivo*, i.e. in cells, or in vitro, which will allow the high throughput analysis of protein functions.

US 6,280,977 B1 describes a rather complex method for producing transcriptionally active DNA molecules comprising PCR amplification of said DNA molecule in the presence of two additional DNA fragments (F1 and F2) as well as four primers (P1-P4). Thereby, F1 comprises a promoter sequence, F2 comprises a terminator sequence, P1 is complementary to the 5' end of F1, P2 is complementary to the 5' end of F2, P3 comprises a first region complementary to the 3' end of F1 and a second region complementary to the 5' end of said DNA molecule, P4 comprises a first region complementary to the 3' end of F2 and a second region complementary to the 3' end of said DNA molecule. This method requires the design of a total of four primers and the knowledge of the DNA sequence of interest to be amplified.

US 2004/0091918 A1 describes an amplification-based method for rapidly producing siRNA expression cassettes. However, the method is customized for siRNA expression cassettes and their use in the screening of potential target sequences susceptible to siRNA mediated degradation. Furthermore, there are two primers used during the amplification, wherein one of the primers comprises not only a sequence that is complementary to the 3' end of the promoter sequence but further comprises one or more sequences which are complementary to a sequence encoding a sense and/or antisense sequence of a siRNA molecule along with one or both loop sequences and a terminator sequence. Again, the sequence of the nucleic acid of interest to be comprised in the cassette has to be known for designing the (second) primer.

Li et al. (Li, S., Brisson, M., He, Y., Huang, L. (1997) Delivery of a PCR amplified DNA fragment into cells: a model for using synthetic genes for gene therapy. Gene Therapy 4: 449-454) describe the use of PCR amplified, double stranded DNA fragments as a model for the.double stranded DNA which is to be obtained by total chemical synthesis and used in gene therapy. In this study the expression of both plasmids and corresponding PCR fragments both containing the CAT reporter gene and CMV or T7 promotor, respectively, were compared after liposome-directed transfection into 293 cells. However, expression was only successful by using a dual expression system either in 293-T7 cells, a cell line which produces endogenous T7 RNA polymerase, or in 293 cells, which were co-transfected with a plasmid (or PCR fragment) containing the T7 RNA polymerase.

Therefore, the problem to be solved by this invention was to provide the use of translationally active linear double stranded DNA cassettes in high throughput arrays, wherein said cassettes are obtained by a simple and efficient method to produce DNA cassettes, that are translationally active, and secondly use them in high through put arrays. Thereby, such a method overcomes the limitations of the amplification methods known in the art, such as the use of more than two primers, complex primer design and knowledge of the nucleic acid sequence of interest for the primer design, as well as the known expression systems for such DNA cassettes, such as dual expression with polymerases.

The problem is solved by the present invention by the uses and kit as claimed in the appended claims.

In particular, the problem is solved by the present invention by the use of a translationally active linear double stranded DNA cassette in a high throughput array, wherein said DNA cassette is obtained by a method comprising PCR amplification of a DNA sequence from an expression vector, wherein the DNA sequence is selected from the group containing cDNA, open reading frame or gene, said method further comprising the use of
(i) a first forward primer complementary to the vector region upstream of the DNA sequence at or near the 5' end of the promoter sequence, and
(ii) a second reverse primer complementary to the vector region downstream of the DNA sequence at or near the 3' end of the termination sequence,
wherein the translationally active linear double stranded DNA cassette generated comprises the promoter, the DNA sequence, and the termination sequence.

In an embodiment, not forming part of the present invention, the method of the invention further comprises the steps of
- transfecting cells with the translationally active DNA cassettes, and
- expressing the molecule that is encoded by the translationally active DNA cassette.

It is preferred that the DNA sequence of interest is a cDNA that codes for a protein of interest or an open reading frame in 5' to 3' orientation.

Examples for proteins of interest are green fluorescent protein (GFP), RNA binding proteins, such as HuR, interferon-stimulated gene products, such as ribonuclease L, therapeutic proteins, growth factors, and cytokines, tissue inhibitor of metalloproteinase 1 (TIMP), tristetraprolin (TTP) and 5' nucleotidase (NT5C3).

It is further preferred that the vector is selected from the group containing a plasmid, a viral-based vector or a vector contained in bacterial or eukaryotic cells or an expression library. Vectors are known in the art. Preferred vectors are expression vectors containing CMV promoter, such as vectors of the pcDNA series, e.g. pcDNA3 (Invitrogen, Inc.), gWiz (Gene Therapy Systems, Inc.), and pIRES vectors (Clontech, Inc.).

Suitable vectors of the present invention are constitutively expressed, inducible, repressed or can be regulated.

According to the invention, preferably the promoter is eukaryotic and the termination site is a poly A sequence containing a polyadenylation signal. Eukaryotic or mammalian promoters and terminators are known in the art. However, preferred eukaryotic promoters are the CMV promoter, SV40 promoter, and RSV promoter. Preferred eukaryotic terminators are bovine growth factor (BGH) poly A site, SV40 poly A, and HSK poly A.

Preferably, the promoter is prokaryotic and the termination sequence is a prokaryotic termination sequence. Prokaryotic promoters and terminators are known in the art. However, preferred prokaryotic promoters comprise the Shine-Dalgarno sequence. Preferred prokaryotic promoters are the T3 promoter, T7 promoter and SP6 promoter. Preferred prokaryotic terminators are T7 terminator, T3, and SP6 promoter terminators.

The preferred promoter can be any promoter, mammalian or bacterial, with or without upstream or downstream enhancing or intron elements.

In the art, methods for transfecting cells with DNA/vectors are well known. For instance, expression vectors are brought into cells by using a variety of DNA transfection methods including, but not limited to, calcium phosphate coprecipitation, electroporation and cationic liposome-mediated transfection (e.g., lipofection). For the latter reagents, such as lipofectinamine (Invitrogen), are commercially available.

At least one of the primers can be modified at the 5' end to enhance transfection and/or expression of the translationally active DNA cassette inside cells.

Thereby, a preferred modification is selected from the group containing phosphorylation, amino, cholesterol, peptide nucleic acids (PNA), and hairpin structures.

In an embodiment, not forming part of the present invention, the method of the invention further comprises the steps of
- synthesizing double-stranded RNA from the translationally active DNA cassettes
- digesting the resultant RNA with RNAse III or dicer enzyme, and
- generating a siRNA population.

It is especially preferred, that the DNA cassettes of the invention are used in a high throughput array.

Thereby, it is preferred, that the DNA cassette is lyophilized with or without a transfection reagent.

It is further preferred, that cells in culture medium are added directly to the arrays.

It is especially preferred, that the DNA cassettes of the invention are used in a functional assay array.

Thereby, it is preferred, that the DNA cassette is recovered by an aqueous solution and transferred into another array or vessels for functional assays.

The DNA cassettes of the invention can be used in an in vitro transcription and translation system.

Thereby, it is preferred, that the DNA cassette is used for preparing proteome arrays from cells, tissues or from cells of a subject to treatment.

It is further preferred that the DNA cassettes are used for preparing proteome arrays in a transfection-ready format.

In another preferred embodiment of the present invention a kit is provided comprising a 96-well plate, 384-well plate or another array, containing at least one of the translationally active DNA cassettes according to the invention and comprising at least one of the following: (a) a buffer solution for eluting the DNA, (b) transfection reagent, and (c) data sheet.

The present invention describes a simple and efficient way to produce translationally active DNA cassettes by using PCR amplification to generate linear DNA with correct orientation (5' to 3') comprising a promoter, a DNA of interest, such as a cDNA, gene or open reading frame, and a termination sequence, such as a polyA site.

The invention describes a method wherein linear double stranded DNA molecules are generated from an expression vector or expression library by the process of the polymerase chain reaction (PCR) using two primers. The DNA product is capable of expressing the protein when transfected into living cells. The invention also describes the construction of an array containing the produced DNA, preferably in a lyophilized form for room temperature transport convenience. The linear DNA contains the minimum on necessary expression information, i.e. a promoter, a DNA sequence of interest (i.e. gene, cDNA, open reading frame (ORF)), and a termination sequence (e.g., polyA site), but lacks other sequences that would allow replication or stable transfection into cells.

The DNA is generated for use in an array format, e.g. in 96-well or 384-well plates in a commercial setting, but the arrays are non-reusable, or at least difficult to reuse. The arrays can be used directly, e.g. cells are added directly along with the appropriate transfection reagent. Alternatively, the DNA in the arrays/plates can be eluted by water, sample buffer or medium and then added onto another plate or an array containing cells.

### BRIEF DESCRIPTION OF DRAWINGS

**Figure 1** *Example scheme of producing mammalian translationally active DNA cassettes as linear double-stranded DNA*. (see Example 1)
**Figure 2****.** *Amplification and production of translationally and expressionally active linear DNA cassettes.*
   Lanes 1 to 6 correspond to the products of six PCR amplification reactions (see Example 2), wherein the expected sizes are ∼ 2.5 kB (PCR product 1), ∼ 2.9 kB (PCR product 2), - 2.2 kB (PCR product 3), ∼ 1.3 kB (PCR product 4, control), ∼ 2.6 kB (PCR product 5), 3.1 kB (PCR product 6).
**Figure 3****.** *Transfection and fluorescence of GFP translation cassettes*.
   HEK293 cells (3 x 10⁴ per well) in 96-well plates were transfected with 600 ng, 200 ng, and 67 ng of purified PCR products of Example 2. The GFP fluorescence intensity in HEK293 cells is shown when transfected with vector containing GFP (panel A) or with the GFP expression DNA cassette using 50 ng of purified PCR product, but without end modification (panel B), see also Example 3.

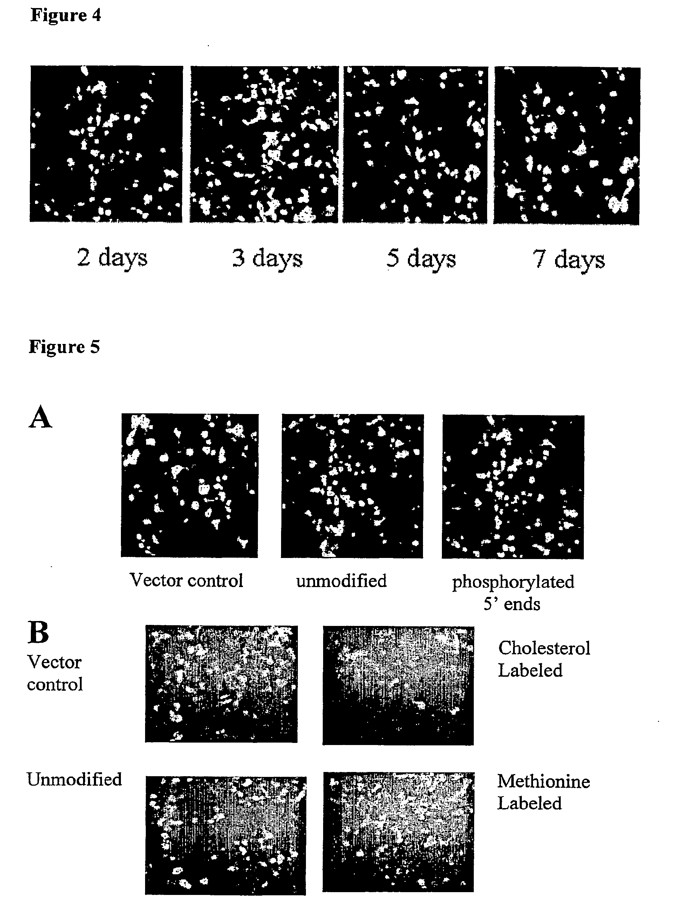

**Figure 4****.** *Expression persistence of GFP translation cassettes*.
   HEK293 cells (3 x 10⁴ per well) in 96-well plates were transfected with 100 ng of purified PCR products containing GFP insert. The GFP fluorescence intensity in HEK293 cells at different time points, up to day 7, is shown.
**Figure 5****.** *Effect of modifications on GFP expression*.
   HEK293 cells (3 x 10⁴ per well) in 96-well plates were transfected with 100 ng of purified PCR products containing GFP insert. The GFP fluorescence intensity in HEK293 cells at 2 days following transfection is shown. Panel A shows the GFP expression using GFP vector, using GFP PCR products without modifications, and using GFP PCR products generated from primers that were 5' modified with phosphate group. Panel B shows the GFP expression using GFP vector, using GFP PCR products without modifications, and using GFP PCR products that were 5' modified with cholesterol or methionine.

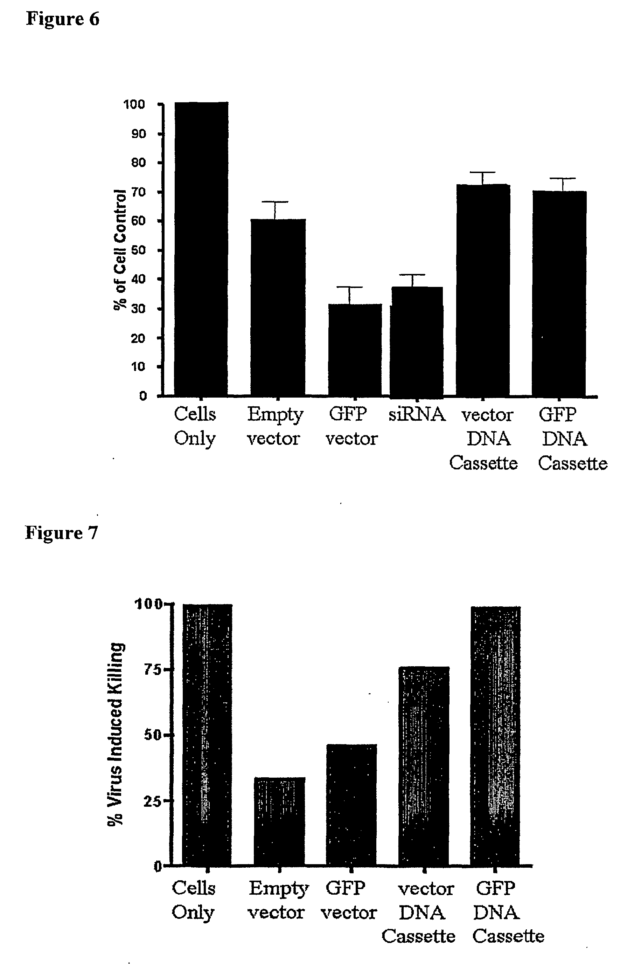

**Figure 6****.** *Effects of translationally active DNA cassettes and of vectors on cellular stress*.
   HEK293 cells (3 x 10⁴ per well) in 96-well plates were transfected (as described in Example 3) with equal amounts (∼100 ng) of pcDNA 3.1 vectors, GFP gWiz vector, PCR products generated from pcDNA 3.1 empty vector, and PCR product generated from GFP gWiz vector. Cellular viability and growth were measured after 20 hours (see Example 6). Data are presented as % of cell control (mean ± SEM) using 6 replicate readings.
**Figure 7****.** *Effects of translationally active DNA cassettes and of vectors on IFN response*.
   HEK293 cells (3 x 10⁴ per well) in 96-well plates were transfected (as described in Example 3) with equal amounts (∼100 ng) of pcDNA 3.1 vectors, GFP gWiz vector, PCR products generated from pcDNA 3.1 empty vector, and PCR product generated from GFP gWiz vector. After 20 hours, cells were challenged with 10⁴ plaque forming units of encephalo myocarditis virus (EMCV). After 18 hour incubation, cellular viability was measured (see Example 7). When IFN response existed, reduction in cell killing is noted. Data are presented as % of virus control (100% killing of cells).

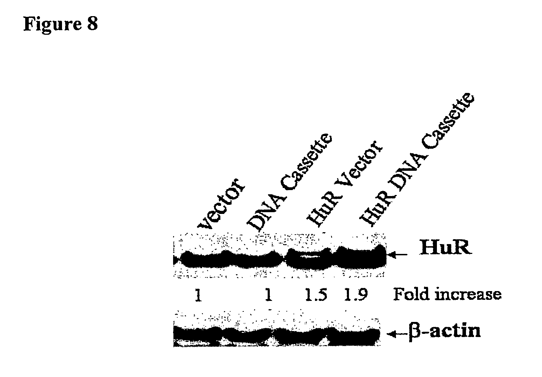

**Figure 8****.** *Expression of HuR protein using translationally active DNA*.
   HEK293 cells (3 x 10⁴ per well) in 96-well plates were transfected, as described in Example 3, with equal amounts (∼100 ng) of pcDNA 3.1 vectors, PCR products generated from pcDNA 3.1 empty vector, pcDNA 3.1 harboring HuR cDNA, and PCR product generated from HuR pcDNA 3.1. After 24 hours, proteins were extracted and analyzed by Western blot using antibodies specific to HuR protein (see Example 8).

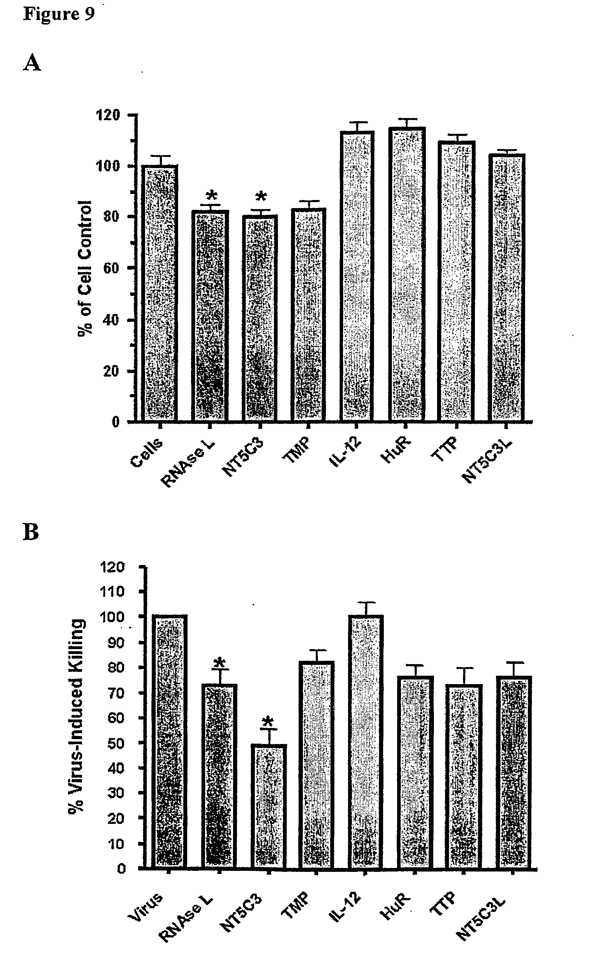

**Figure 9****.** *Production and use of high-throughput functional arrays containing translationally active DNA*.
   A number of cDNAs were cloned into pcDNA 3.1 and PCR products were generated (as described in Examples 1 and 2). The PCR products were spotted on 96-well plates as an array of translationally active DNA suitable for use with high throughput technologies and assays. The DNA with lipofectinamine 2000 transfection reagent were transferred into 96-well plates containing HEK293 cells (4 x 10⁴ cell/ml) in a replicate of 8. After 24 hours, the plates were used for either an anti-cellular (panel A) or an anti-viral assay (panel B) in 96-well plate array, see Example 9. The gene products used were ribonulcease L (RNAse L), 5' nucleotidase (NT5C3), tissue inhibitor of metalloproteinase 1 (TIMP), interleukin-12 (IL-12), HuR, tristetraprolin (TTP) and NT5C3L. The stars* refer to significant changes.

### DETAILED DESCRIPTION OF THE INVENTION/ PREFFERED EMBODIMENTS

The present invention describes a simple and high-throughput method for the construction of translationally active linear PCR products using two primers complementary to two sites in an expression vector, preferably a mammalian expression vector. The translation cassette is suitable for mammalian expression after transfecting it into mammalian cells using conventional transfection techniques. The method allows the use of array type formats such as a 96-well, a 384-well format (e.g. plate) or any other formats, such as cell microarrays.

The present invention provides the uses and kit as claimed in the appended claims.

The use of non-vector bearing DNA molecules allows the manufacturing of non-reusable, one-way commercial products, because the DNA cannot or is difficult to be reused. This is one of the main goals of this approach.

The vector with the DNA sequence of interest is constructed by any amplification process, e.g., by PCR, of the DNA sequence of interest, such as specific coding region or cDNA, with or without the use of 5' or 3' tags such as GST, HA or fusion proteins, such as GFP.

The method is preferably suitable for individual expression vectors or an expression library. An expression library can be substracted, normalized, or enriched in any gene or protein family

The vector with the DNA sequence of interest preferably originates from an expression library. Thus, vectors do not only harbor a defined DNA sequence of interest, e.g. cDNA, as those obtained by PCR of known or hypothetical genes. But also vectors with novel DNA sequences of interest, e.g. cDNAs, that are generated from alternatively spliced or rare genes in expression libraries are sources of the template. In case of an expression library, the preferred embodiment is an expression library with a reduced frequency of abundant and housekeeping genes, such as normalized, substracted, and enriched libraries.

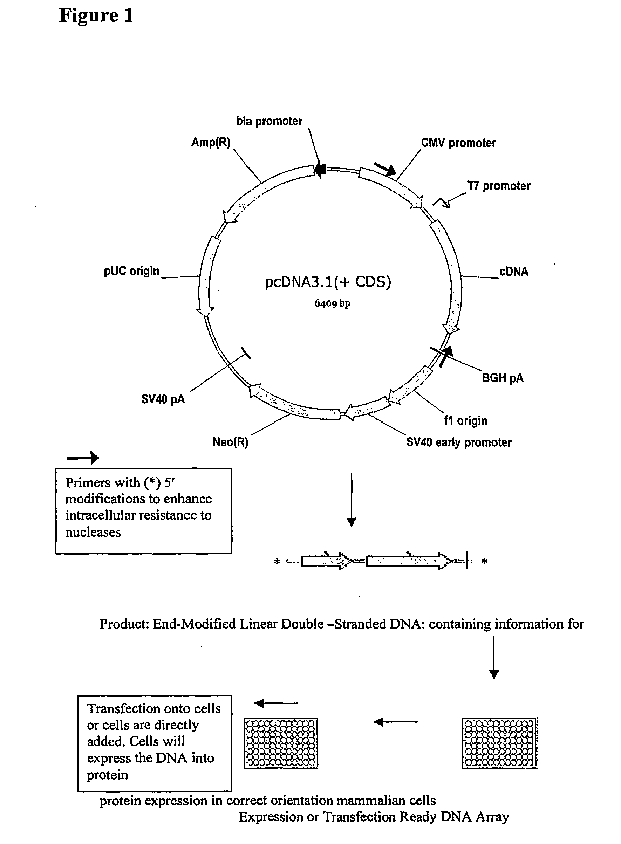

Figure 1 shows an example scheme of the method. The translationally active DNA cassettes are produced by polymerase chain reaction (PCR) using two primers.

Though the preferred embodiment is a PCR-based method, the invention may be practiced or modified based on other DNA formation or amplification methods known in the art, such as the helicase-dependent isothermal amplification (Vincent M, Xu Y, Kong H (2004) Helicase-dependent isothermal DNA amplification. EMBO Rep. 8: 795-800.).

The PCR method utilizes one pair of primers. The first primer is complementary to a region upstream of the DNA sequence of interest (gene, cDNA, ORF or coding region) and specifically complementary to the region comprising the promoter. Whereas the second primer is complementary to a region downstream of the end of the DNA sequence of interest (gene, cDNA, ORF or coding region) and specifically complementary to a termination sequence, e.g. the poly A site. The poly A site acts as a terminator of the transcription of the DNA sequence of interest. If the poly A site is already part of the DNA sequence of interest the first polyA site can act as the terminator. Customized expression vectors are engineered to contain an artificial Poly A site, such as a SV Poly A site.

The preferred site of the first primer is at or near (preferably upstream) the 5' end of the promoter. The promoter can be any promoter, mammalian or bacterial, with or without upstream or downstream enhancing or intron elements.

The preferred site of the second primer is the 3' end of the termination sequence, such as the polyA site.

In order to protect the resultant PCR products from degradation by intracellular nucleases and, therefore, enhance expression and translation of the cassettes, one or both of the primer 5' ends may be modified by any method that protects these ends. There is a variety of methods available, for example, as part of the primer synthesis services by a number of companies, such as Sigma-GenoSys, prooligo, Qiagen, MWG, and many others. Such modifications include but are not limited to phosphorylation, amino group, cholesterol moieties, peptide nucleic acids (PNA), and hairpin structures.

Modifications in the primers may also allow efficient transfection, such as the 5' end moiety of cholesterol, liposome, or any other lipid moiety to allow better transfection through the lipid cellular membrane. Modifications can also be carried out on the PCR products themselves to allow for conjugation to the 3' end.

The resultant PCR products will contain a promoter, the DNA sequence of interest, and a termination sequence, such as a polyA site. Since the linear DNA is ready to transfect cells; cells will take up the cassettes, express the gene, and translate the protein using the cells machinery.

In one embodiment, the DNA cassettes obtained by the method according to the invention can be used in a cell microarray format. The invention is different from the method disclosed in Sabatini et al. (US patent application 2003/0228694), because the method of the present invention uses instead of entire expression vectors (as in Sabatini *et al*.) only linear translationally active DNA cassettes without the rest of the vector backbone. In addition, the method according to the invention utilizes standard transfection techniques and does not require a carrier protein or glass slides (as in Sabatini *et al*.). Furthermore, the method according to the invention does not depend on the "reverse transfection", a primary claim in Sabitini *et al*..

In a preferred application of the invention, transfected living cells or transfected non-living cells, e.g., fixed cells, are used in an array type for expressing membrane proteins to study or capture interacting molecules, such as antibodies, interacting proteins, peptides, or other analogues thereof.

The use of the present invention in array format allows for high-throughput screening of lead structures in target specificity of protein-small molecule interactions, and potentially for characterizing protein-enzyme and protein-antibody interactions.

The present invention in array format can be used to identify potential drug targets by functionally characterizing large numbers of gene products in cell-based assays, evaluating the specificity of drug lead structures; and identifying binding proteins for drugs of unknown mechanism of action or for lead structures identified in phenotype-based assays.

The mammalian translationally active DNA cassette of the invention can be used in an *in vitro* transcription/translation system, if the forward primer is complementary to e.g. the T7 promoter in the vector. For example, the vertical arrow designated as T7 promoter in the expression vector pcDN3.1 (see Figure 1). The vector can be any vector as long as it contains the T7 promoter.

Prokaryotic translation cassettes contain the prokaryotic promoter, such as T3, T7, and SP6 promoter sequence, the DNA sequence of interest , and termination signal.

In another embodiment, not forming part of the present invention, the prokaryotic translation cassette is used in *in vitro* transcription and translation systems. The phage promoter, such as T7, T3, or SP6, drives the transcription reaction by the RNA polymerase. In case of this embodiment, the forward specific primer is complementary to the phage promoter sequence. The *in vitro* translation systems should contain crude cellular extracts, providing all of the essential components necessary to translate the protein, such as ribosomes and tRNAs. There are several lysate systems, such as rabbit reticulocytes, wheat germ, or from a strain of *Escherichia coli* (F. Movahedzadeh et al. In vitro transcription and translation, in Methods in Molecular Biology, V. 235, N. Casali, A. Preston, Eds., Totowa, NJ: Humana Press, p. 247-55; T. Lamla et al. (2001) The cell-free protein biosynthesis - applications and analysis of the system, Acta Biochim Pol, 48: 453-65.). Several companies provide *in vitro* transcription and translation systems, including high-throughput modes, such as Qiagen, Roche, Ambion, Promega, and Novagen.

Use in a mammalian expression system, including *in vitro* and in cells, requires a ribosome binding site, such as Kozak sequence CACCATGG (Kozak, M. (1987) An analysis of 5'-noncoding sequences from 699 vertebrate messenger RNAs. Nucleic Acids Res. 15: 8125-8148.) at least six nucleotides preceding the ATG initiator codon enhance translation (Kozak, supra). This may be provided in the source template, vector, or part of the full-length cDNA (upstream of ATG start site of the cDNA). In many cases, the 5' upstream sequences of the ATG start site can also supplant the typical Kozak sequences (Khabar KS, Dhalla M, Bakheet T, Sy C, al-Haj L. (2002) An integrated computational and laboratory approach for selective amplification of mRNAs containing the adenylate uridylate-rich element consensus sequence. Genome Res. 6: 985-95.).

Use in bacterial expression systems requires the use of the bacterial Shine-Dalgarno sequence as the ribosome binding site (Shine, J., and Dalgarno, L. (1974) Proc. Natl. Acad. Sci. USA 71: 1342-1346). This is supplied as part of the vector or can be incorporated as part of the forward primer. Also, this system may require a terminator signal, which can be supplied in the reverse primer if it does not exist in the vector template.

The invention described here is suitable to produce focused functional arrays by preparing the proteome representation of specific families of genes, diseases, or research areas. For example, a cytokine functional array or kit comprises translationally active cassettes derived by PCR, wherein the template is an expression vector harboring a cytokine. A chemokine functional array or kit comprises translationally active cassettes derived by PCR, wherein the template is an expression vector harboring a chemokine. An angiogenesis functional array or kit comprises translationally active cassettes derived by PCR, wherein the template is an expression vector harboring a gene implicated in the angiogenesis process. A lung cancer functional array or kit comprises translationally active cassettes derived by PCR, wherein the template is an expression vector harboring a gene implicated in lung cancer.

Another major advantage of the method according to the invention is that the use of translationally active DNA cassettes resulted in reduced stress on cells in comparison the use of to vectors or siRNA. It is known that vectors and siRNA activate the IFN response and cause non-specific reduction in cellular growth of the cells (Sledz, C.A., Holko, M., de Veer, M.J., Silverman, R.H., Williams, B.R. (2003) Activation of the interferon system by short-interfering RNAs. Nat. Cell Biol. 9: 839-9.; Jackson, A.L., Linsley, P.S. (2004) Noise amidst the silence: off-target effects of siRNAs. Trends Genet. 11:521-4.). These effects can dramatically influence many of the results obtained by siRNA screening.

### EXAMPLES

The following examples are intended for illustration purposes only, and should not be construed as limiting the scope of the present invention in any way.

### Example 1: Scheme of producing mammalian translationally active DNA cassettes as linear double-stranded DNA. (see Figure 1)

The method starts with amplifying the region flanking the gene of interest, e.g., cDNA, open reading frame, or coding region, from an expression vector, e.g. pcDNA3.1, using two primers. The amplified region should contain the minimal promoter sequences needed for transcription of the DNA and termination sequences, such as the bovine growth factor (BGH) poly A site. The forward (5') primer targets the region upstream of the DNA sequence of interest at the promoter site while the reverse (3') primer targets the region downstream of the DNA sequence of interest at the BGH poly A site. The resultant PCR product, called mammalian translation cassettes or protein expression cassettes, will be linear double-stranded DNA containing the information that are necessary to transcribe and translate the protein in correct orientation of the cDNA. The star * refers to the modification in the primer 5' ends in order to combat intracellular mRNA degradation. The translation cassettes are purified by column chromatography. The mammalian translation cassettes are arrayed in a 96-well or 384-well plate or any other formats that can be directly used as transfection array (i.e., by adding cells directly on the arrays) or transferred to other culture vessel arrays of choice that contain the cells of interest. Cells will express the protein in each well and the phenotype can be studied in any cell-based or functional assay.

### Example 2: Production of expressionally active DNA cassettes

Three plasmids were used, an expression vector (gWiz vector) that contains green fluorescent protein (GFP) (plasmid 1), a pcDNA 3.1 vector that contains the RNA binding protein HuR (plasmid 2), and a pcDNA 3.1 vector that contains no insert (plasmid 3). Several pairs of primers were used. Three pairs of primers were used with the following sets of primers:
+CMV prom, CMV prom,
BGH poly, +BGH poly.

The first, forward (5') primers are complementary to the beginning of the CMV promoter region or upstream of the promoter, while the second, reverse (3') primers are complementary to the BGH polyA site or downstream of this site. Primer sequences are shown in Table 1.

| **Table 1: Primers Sequences** |
|---|
| A) Forward Primers: |
| **+CMV prom** |
| 5' CGA AAA GTG CCA CCT GAC GT |
| This primer is complementary to |
| -382 CMV promoter in g Wiz vector (plasmid 1) and |
| -232 CMV promoter in pcDNA 3.1 vector (plasmids 2 and 3). |
| |
| **CMV prom** |
| 5' CG GTT GAC ATT GAT TAT TGA CTA GT |
| The additional two 5'nucleotides increase the primer stability, while the rest of the sequence is the beginning of the CMV promoter in plasmids 1, 2, and 3. |
| |
| B) Reverse Primers: |
| **BGH poly** |
| 5' CCA TAG AGC CCA CCG CAT |
| This primer targets the end of the BGH polyA site and contains the termination site for plasmids 1, 2, and 3. |
| |
| **+BGH poly** |
| 5' CGG GTC AAT TCT TCA GCA C |
| This primer is complementary to +100 downstream of the end of the BGH site and is specific for plasmid 2. |

Six PCR amplifications were carried out using a mixture of *Taq* and *Pfu* polymerase in a 100 µl reaction with the following cycle conditions:
- 95°C for 12 min. (to activate hot start polymerases),
- 35 cycles of: 94°C, 1 min., 52°C, 1 min., 72°C, 4 min., and
- final extension at 72°C for 7 min.

The PCR products were purified using Qiagen PCR purification columns to eliminate the primers, small PCR products, buffer, and enzymes. The PCR products were eluted in sterile water.

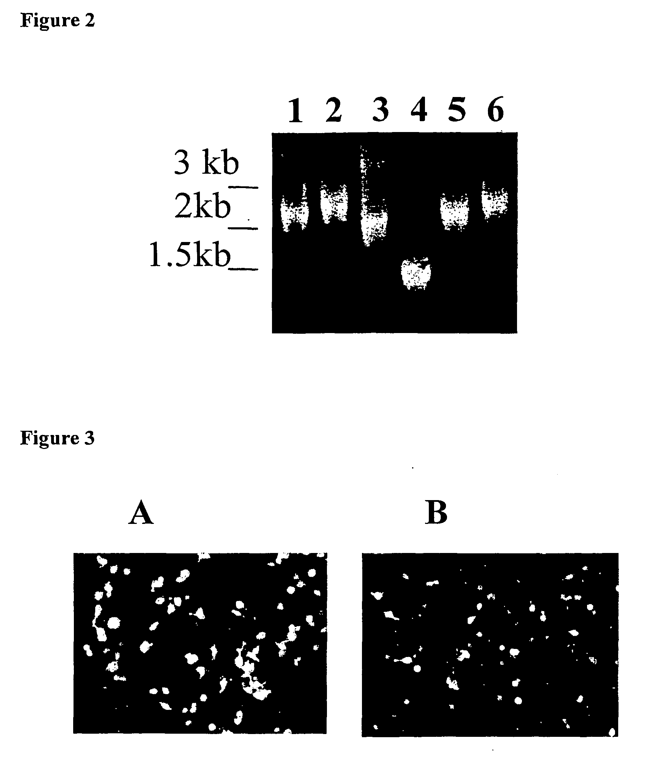

From each sample an 8 µl aliquot was run on a 1.2 % agarose gel and visualized by ethidium bromide under UV light (Figure 2).

The size of the expected PCR product are as follows:

| | |
|---|---|
| **PCR product 1:** | - source: plasmid 1, GFP cassette |
| | - primers: CMV prom and +BGH poly |
| | - size ∼ 2.5 Kb; |
| **PCR product 2:** | - source: plasmid 1, GFP cassette |
| | - primers: +CMV prom and +PGH poly |
| | - size ∼ 2.9 Kb; |
| **PCR product 3:** | - source: plasmid 2, HuR cassette |
| | - primers: +CMV prom and +BGH poly |
| | - size ∼ 2.2 Kb; |
| **PCR product 4:** | - source: plasmid 3 (control pcDNA 3.1), no insert |
| | - primers: / |
| | - size ∼1.3 Kb; |
| **PCR product 5:** | - source: plasmid 1, GFP cassette, |
| | - primers: +CMV prom and +BGH poly |
| | - size: ∼ 2.6 Kb; |
| **PCR product 6:** | - source: plasmid 1, GFP cassette, |
| | - primers: +CMV prom and BGH poly |
| | - size: 3.1 Kb. |

### Example 3: GFP DNA transfection and expression

HEK293 cells were grown at standard culture conditions (37°C, 5% CO₂) in RPMI 1788 medium supplemented with 10% FBS and antibiotics (GIBCO BRL, Gaithersburg, MD). 3 x 10⁴ cells per well in 96-well plates were transfected with 600 ng, 200 ng, and 67 ng of the purified PCR products of Example 2. Transfections were performed in serum-free medium using LipofectinAmine 2000 (Gibco) for 5 h, followed by replacing medium with serum-supplemented medium.

In general, GFP was illuminated with a fluorescence microscope (optimum excitation wavelength: 488 nm and emission wavelength: 503 nm). Images were captured as TIFF file and converted to gray scale and mean intensity was quantified using NIH image analysis software. Alternatively, the number of GFP positive cells was visually (qualitatively) assessed.

The fluorescence of 67 ng transfection concentration was the highest followed by 200 ng and then 600 ng (see Table 2). There were also differences among the different PCR products.

Figure 3 shows the GFP fluorescence in HEK293 cells when transfected with vector containing GFP (panel A) or with the GFP expression DNA cassette using 50 ng of purified PCR product but without end modification (panel B). The main fluorescence intensity of the cells transfected with translationally active cassettes is lower than of those transfected with vector. Thus, modifications of the 5' ends of the primers are strongly suggested as described in the invention.

| **Table 2: Relative efficiency of GFP fluorescence** | | | |
|---|---|---|---|
| | 600 ng | 200 ng | 67 ng |
| PCR product 1 | ++ | +++ | ++++ |
| PCR product 2 | + | ++ | ++ |
| PCR product 5 | +++ | +++ | +++ |
| PCR product 6 | + | + | ++ |
| Vector control | +++++ | | |

### Example 4: Persistence of GFP translationally active (PCR) cassettes.

HEK293 cells, 3 x 10⁴ cells per well in 96-well plates, in RPMI growth medium (10% serum), were transfected with 100 ng of the purified PCR product 1 of Example 2. Transfections were performed in serum-free medium using LipofectinAmine 2000 (Gibco) and mixed together with HEK cells for 5h, followed by replacing medium with serum-supplemented medium. The fluorescence was visualized as described above. We observed, that the expression from the PCR products was sustained as long as the cells remained healthy (up to 7 days in our hands), the reason for this might be that the PCR products are gentle on the cells compared to vectors.

Figure 4 shows the GFP fluorescence in HEK293 cells when transfected with PCR product from day 2 to day 7. No loss of expression was observed, even at day 7.

### Example 5: Effect of 5' modification on GFP expression.

In order to assess the effect of 5' end modification of the primers used for amplification, primers were used that were 5' end modified with a phosphate group, cholesterol, and methionine. Transfection conditions and fluorescence measurement, see above.

Figure 5 shows the results to two independent experiments employing modifications of the 5' primers. The sequences of the primers are the same as CMV Prot and PGH Poly (see Table 1). There were no significant differences with modifications with the various groups except with methionine. The mean intensity was 97 arbitrary units for PCR products generated from 5' methionine labeled primers compared to a mean intensity of 82 arbitrary units with unlabeled products.

### Example 6: Anti-cellular effects of the translationally active DNA cassettes.

HEK293 were transfected, as described above in Example 3, with equal amounts (∼100 ng) of pcDNA 3.1 vectors, GFP gWiz vector, PCR products generated from pcDNA 3.1 empty vector, and PCR product generated from GFP gWiz vector, and siRNA (Proligo, France; 10 nM) were added to HEK 293 cells in 96-well microplate. Cellular viability and growth was measured after 20 hours with a microplate luminometer using the Promega's Cell Titer-Glo™ luminescent cell viability assay (Figure 6). Data are presented as % of cell control using mean of 6 replicates with SEM. Transfection with PCR products caused less non-specific effects, such as stress or toxicity, to the cells as seen with effect on cell viability and growth. For example, there were only 30% reductions in cell viability/growth when cells were transfected with the PCR products compared to 60-70% reduction in cell viability/growth when transfected with the plasmids or siRNA. This indicates that the translationally active DNA cassettes according to the present invention are less stressful than vectors themselves. The described approach thus leads to significant reduced stress on cells as compared to vectors and siRNA.

### Example 7: Activation of the IFN response in cells treated with vectors and translationally active DNA cassettes.

HEK293 cells (3 x 10⁴ per well) in 96-well plates were transfected, as described above in Example 3, with equal amounts (∼100 ng) of pcDNA 3.1 vectors, GFP gWiz vector, PCR products generated from pcDNA 3.1 empty vector, and PCR product generated from GFP gWiz vector. After 20 hours, cells were challenged with 10⁴ plaque forming units of encephalo myocarditis virus (EMCV). After 18 hour incubation, cellular viability was measured with a microplate luminometer using the Promega's Cell Titer-Glo™ luminescent cell viability assay. When IFN response existed, reduction in cell killing is noted. Data are presented as % of virus control (100% killing of cells).

A major limitation of siRNA and vectors is the induction of IFN response which may influence the findings of the functional studies of gene products directed by siRNA or vectors. In this experiment it was found that the translationally active DNA caused less activation of IFN response, as measured by the ability to reduce EMC virus-induced killing, as seen in Figure 7.

### Example 8: Over-expression of selected gene products with vectors and translationally active DNA cassettes.

To further assess the suitability of the translationally active DNA cassettes in inducing ectopic expression of selected gene products, the following were used: pcDNA 3.1 vector and pcDNA 3.1 containing HuR cDNA, and PCR products generated from them as translationally active DNA cassettes (Example 2, PCR Product 3).
HEK293 cells (3 x 10⁴ per well) in 96-well plates were transfected, as described in Example 3, with equal amounts (∼100 ng) of pcDNA 3.1 vectors, PCR products generated from pcDNA 3.1 empty vector, pcDNA 3.1 harboring HuR cDNA, and PCR product generated from HuR pcDNA 3.1. After 24 hours, proteins were extracted, run on an SDS polyacrylamide gel (PAGE), transferred to membranes, and probed with anti-HuR antibodies (Santa Cruze Biotech, USA). Blots were visualized using chemiluminescence. Blots were re-probed with antibodies to beta actin to verify loading and quality of cell lysates.
These Western blotting analysis using specific antibodies to HuR protein showed that the over-expression in HEK293 with the translationally active PCR product is higher (2-fold) than with conventional vector expression (1.5-fold), see Figure 8.

### Example 9. Production and use of high-throughput functional arrays containing translationally active DNA.

A number of cDNAs were cloned into pcDNA 3.1 and PCR products were generated, as described above. The gene products used are ribonulcease L (RNAse L), 5' nucleotidase (NT5C3), tissue inhibitor of metalloproteinase 1 (TIMP), interleukin-12 (IL-12), HuR, tristetraprolin (TTP) and NT5C3L. The PCR products can be easily generated in 96-well or 384-well plates as a high throughput process. The PCR products were spotted on 96-well plates as an array of translationally active DNA suitable for use with high throughput technologies and assays. The microplate acts as an array format with known location of each clone and known identity of each clone.

The translationally active PCR products (100 ng each) with lipofectinamine 2000 transfection reagent were transferred into 96-well plates containing HEK293 cells (4 x 10⁴ cell/ml) in a replicate of 8 wells. After 24 hours, the plates were used for either an anti-cellular (panel A of Figure 9) or anti-viral assay (panel B of Figure 9) as 96-well plate array. The anti-cellular and anti-viral assays were as described in Examples 6 and 7.

This method allowed rapid screening of cDNA with anti-cellular and anti-viral action. RNAse L was used as a positive control cDNA in which its gene product mediates both anti-cellular and anti-viral action (Khabar, K.S., Siddiqui, Y.M., al-Zoghaibi, F., al-Haj, L., Dhalla, M., Zhou, A., Dong, B., Whitmore, M., Paranjape, J., Al-Ahdal, M.N., Al-Mohanna, F., Williams, B.R., Silverman, R.H. (2003) RNase L mediates transient control of the interferon response through modulation of the double-stranded RNA-dependent protein kinase PKR. J Biol Chem. 278(22): 20124-32; Zhou, A,, Paranjape, J.M., Hassel, B.A., Nie, H., Shah, S., Galinski, B., Silverman, R.H. (1998) Impact of RNase L overexpression on viral and cellular growth and death. J Interferon Cytokine Res. 11: 953-61 ). RNAse L was able to reduce cell and virus growth (Figure 9). Other candidate gene products with possible anti-cellular and anti-viral action, most notably NT5C3, were also found This example shows the utility of the translationally active DNA cassette arrays according to the present invention.

SUMMARY of unique features of the translationally active DNA cassettes and their arrays:
1. High-throughput application.
2. Low cost and simple to produce.
3. Compatibility with existing cell-based assays and detection equipment.
4. DNA Cassettes are not toxic, stressful, and may not induced IFN response.
5. Overall transfection is satisfactory and sustained. Expression levels are moderate and not overly artificial which may cause non-specific effects.

The features disclosed in the foregoing description and in the claims may, both separately and in any combination thereof, be material for realizing the invention in diverse forms thereof.

## Claims

1. Use of a translationally active linear double stranded DNA cassette in a high throughput array,
wherein said DNA cassette is obtained by a method comprising PCR amplification of a DNA sequence from an expression vector, wherein the DNA sequence is selected from the group containing cDNA, open reading frame or gene, said method further comprising the use of
(i) a first forward primer complementary to the vector region upstream of the DNA sequence at or near the 5' end of the promoter sequence, and
(ii) a second reverse primer complementary to the vector region downstream of the DNA sequence at or near the 3' end of the termination sequence,
wherein the translationally active linear double stranded DNA cassette generated comprises the promoter, the DNA sequence, and the termination sequence.

2. Use according to claim 1, wherein the DNA cassette is lyophilized with or without a transfection reagent.

3. Use according to claim 1, wherein cells in culture medium are added directly to the arrays.

4. Use of a DNA cassette as obtained in claim 1 in a functional assay array.

5. Use according to claim 4, wherein the DNA cassette is recovered by an aqueous solution and transferred into another array or vessels for functional assays.

6. Use of a DNA cassette as obtained in claim 1 for preparing proteome arrays from cells or tissues.

7. Use of a DNA cassette as obtained in claim 1 for preparing proteome arrays in a transfection-ready format.

8. A kit comprising a 96-well plate, 384-well plate or another array, containing at least one of the translationally active DNA cassettes as obtained in claim 1 and comprising at least one of the following: (a) a buffer solution for eluting the DNA, (b) transfection reagent, and (c) data sheet.

## Patentansprüche

1. Verwendung einer translationsaktiven linearen doppelsträngigen DNA-Kassette in einem Hochdurchsatz-Array,
wobei die DNA-Kassette durch ein Verfahren erhalten wird, umfassend PCR-Amplifizierung einer DNA-Sequenz von einem Expressionsvektor, wobei die DNA-Sequenz ausgewählt ist aus der Gruppe, enthaltend cDNAs, offene Leserahmen oder Gene, wobei das Verfahren weiterhin die Verwendung von
(i) einem ersten Forward-Primer, komplementär zur Vektorregion, die stromauf von der DNA-Sequenz bei oder nahe dem 5'-Ende der Promotersequenz liegt, und
(ii) einem zweiten Reverse-Primer, komplementär zur Vektorregion, die stromab von der DNA-Sequenz bei oder nahe dem 3'-Ende der Terminationssequenz liegt,
umfasst,
wobei die erzeugte translationsaktive lineare doppelsträngige DNA-Kassette den Promoter, die DNA-Sequenz und die Terminationssequenz umfasst.

2. Verwendung nach Anspruch 1, wobei die DNA-Kassette mit oder ohne Transfektionsreagenz lyophilisiert ist.

3. Verwendung nach Anspruch 1, wobei Zellen in Kulturmedium direkt zu den Arrays zugegeben werden.

4. Verwendung einer DNA-Kassette, wie in Anspruch 1 erhalten, in einem Funktionsassay-Array.

5. Verwendung nach Anspruch 4, wobei die DNA-Kassette mit einer wässrigen Lösung wiedergewonnen und in einen anderen Array oder Gefäße für Funktionsassays übertragen wird.

6. Verwendung einer DNA-Kassette, wie in Anspruch 1 erhalten, zum Herstellen von Proteomarrays aus Zellen oder Geweben.

7. Verwendung einer DNA-Kassette, wie in Anspruch 1 erhalten, zum Herstellen von Proteomarrays in einem transfektionsfertigen Format.

8. Kit, umfassend eine 96-Napfplatte, 384-Napfplatte oder einen anderen Array, enthaltend wenigstens eine der translationsaktiven DNA-Kassetten, wie in Anspruch 1 erhalten, und umfassend wenigstens eines der folgenden: (a) eine Pufferlösung zum Eluieren der DNA, (b) Transfektionsreagenz, und (c) ein Datenblatt.

## Revendications

1. Utilisation d'une cassette d'ADN double brin linéaire active d'un point de vue traductionnel dans une puce haut débit, dans laquelle ladite cassette d'ADN est obtenue par un procédé comprenant une amplification par PCR d'une séquence d'ADN à partir d'un vecteur d'expression, et dans laquelle la séquence d'ADN est sélectionnée dans le groupe contenant un ADNc, un cadre de lecture ouvert ou un gène, ledit procédé comprenant en outre l'utilisation
(i) d'une première amorce sens complémentaire à la région du vecteur située en amont de la séquence d'ADN au niveau ou à proximité de l'extrémité 5' de la séquence promotrice, et
(ii) d'une deuxième amorce antisens complémentaire à la région du vecteur située en aval de la séquence d'ADN au niveau ou à proximité de l'extrémité 3' de la séquence de terminaison,
dans laquelle la cassette d'ADN double brin linéaire active d'un point de vue traductionnel générée comprend le promoteur, la séquence d'ADN et la séquence de terminaison.

2. Utilisation selon la revendication 1, dans laquelle la cassette d'ADN est lyophilisée avec ou sans un réactif de transfection.

3. Utilisation selon la revendication 1, dans laquelle des cellules dans un milieu de culture sont directement ajoutées dans les puces.

4. Utilisation d'une cassette d'ADN telle qu'obtenue dans la revendication 1, dans une puce de dosage fonctionnel.

5. Utilisation selon la revendication 4, dans laquelle la cassette d'ADN est récupérée par une solution aqueuse puis transférée dans une autre puce ou dans des récipients pour des dosages fonctionnels.

6. Utilisation d'une cassette d'ADN telle qu'obtenue dans la revendication 1, pour la préparation de puces à protéome à partir de cellules ou de tissus.

7. Utilisation d'une cassette d'ADN telle qu'obtenue dans la revendication 1, pour la préparation de puces à protéome dans un format prêt pour une transfection.

8. Kit comprenant une plaque à 96 puits, une plaque à 384 puits ou une autre puce, contenant au moins une des cassettes d'ADN actives d'un point de vue traductionnel telles qu'obtenues dans la revendication 1, et comprenant au moins un des éléments suivants : (a) une solution tampon pour éluer l'ADN, (b) un réactif de transfection, et (c) une feuille de données.
